# EUROPEAN PATENT APPLICATION

(11) **EP 4 085 948 A1**
(43) Date of publication of application: **09.11.2022**
(21) Application number: 20757395.7
(22) Date of filing: 10.07.2020
(51) Int. Cl.: A61M 5/32

(54) **SYSTEM FOR REMOVAL AND STORAGE OF CUTTING/PERFORATING INSTRUMENTS**

(30) Priority: 30.12.2019 PT 2019116042
(71) Applicant: Escola Superior de Enfermagem de Coimbra, 3001-901 Coimbra (PT); Instituto Politécnico de Coimbra, 3000-271 Coimbra (PT)
(72) Inventor: SANTOS DINIS PARREIRA, Pedro Miguel, 3045-293 Coimbra (PT); DE SOUSA SALGUEIRO-OLIVEIRA, Anabela De Sousa Salgueiro-oliveira, 3030-175 Coimbra (PT); PEREIRA DE SÁ, Ana Filipa, 3045-441 Ribeira De Frades (PT); FONTOURA DA SILVA, Andreia Filipa, 3850-715 Ribeira De Fráguas (PT); DA SILVA GOMES, Fábio Miguel, 3850-835 Valmaior (PT); DE CARVALHO AGOSTINHO, Luciana, 3000-055 Coimbra (PT); BIDARRA LOPES CARVALHO, Pedro António, 3045-418 Ribeira De Frades (PT); MENDONÇA MARQUES DONATO, Ana Teresa, 3030-428 Coimbra (PT); VALENÇA NEVES, Ana Sofia, 3020-507 COIMBRA (PT); RODRIGUES LEIGO, Beatriz, 3070-728 Praia De Mira (PT); SOUSA JERÓNIMO, Inês Alexandra, 3030-428 Coimbra (PT); PEREIRA DA ROCHA, João Pedro, 4850-052 Caniçada (PT); MENESES ORMONDE CABRITA GRADE, Leonor, 3030-329 Coimbra (PT); OLIVEIRA FRADA, Marta, 3070-541 Seixo (PT); DOS SANTOS PEREIRA MALÇA, Cândida Maria, 3000 COIMBRA (PT); FIGUEIREDO MAROUVO, Pedro, 3030-393 COIMBRA (PT); FERNANDES ALVES, João Carlos, 2435 Casal Dos Bernardos (PT)
(74) Representative: Lourenço Martinho do Rosário, Ana Margarida
(86) International application number: PCT/IB2020/056520
(87) International publication number: WO 2021/136984

(57) **Abstract**

This invention relates to a system intended for the removal and conditioning of sharp-perforating material, which has the primary objective of eliminating the contact between the user and the system, thus avoiding contamination or injury to the user. This invention comprises a base (1) connected to an extendable column (5) and to an arm (2), also comprising a bin (6) and a lid (4). The lid (4) has a separating device (7) incorporated in the lid (4) and this separating device (7) has means of retention and extraction wherein the said means of retention and extraction, when moved downwards, retain the sharp-perforating material and, when moved upwards, the sharp-perforating material is detached and conditioned inside the bin (6).

## Description

### Summary and scope of the invention

This invention relates to a system intended for the removal and conditioning of sharp-perforating material, which has the primary objective of eliminating the contact between the user and the system, thus avoiding contamination or injury to the user.

In the daily practice of healthcare professionals, sharp-perforating materials are commonly used, such as, e.g., needles, scalpels, mandrels, butterflies, etc., which after use are discarded in suitable containers known as sharps bins, hereinafter referred to as bins only, which are then incinerated.

The problem associated with the disconnection and disposal of sharp-perforating materials is based on the need for them to be safely placed on bins, avoiding direct contact by healthcare professionals. This situation often makes it impossible, for example, to continue the clinical procedure previously initiated by the professional, in addition to contributing to a significant increase in the risk of hand contamination or accidental needlestick injury to the healthcare professional. As a typical example, one may think of the common practice in which the healthcare professional needs to perform three administrations of medication to the same person by intravenous, intramuscular and subcutaneous routes. The first administration is followed by the disposal of the needle, i.e., the needle is deposited in the bin. Direct contact with the bin by the healthcare professional involves stopping the clinical procedure for hand hygiene and glove replacement. The workflow is thus interrupted in order to carry out the second administration of medication given the need to directly contacting with the bin for discarding and disposing of the sharp-perforating material (needle) in the bin, and so on. This occurs very often in the daily clinical practice, since many of these medical devices are inserted in different anatomical locations with the aid of mandrels which are then removed and disposed of in bins. Needles/butterflies are also used daily and need to be disconnected from syringes after use.

The conventional systems available for disconnection and disposal of the sharp-perforating material imply the direct contact of the healthcare professional with the bin. The latter must be held with one hand, while the needle that is connected to the syringe is fitted into an integrated structure in the bin, and then an upward movement is made so as to "pull", thus allowing the needle to be disposed of into the bin. The procedure described implies the need for further hand hygiene and replacement of gloves, in addition to increasing the risk of accidental needlestick and also the risk of contamination with medical or organic fluids presents in the sharp-perforating material used. This also contributes to a higher incidence of healthcare-associated infections.

Difficulties as regards the correct disposal also arise due to the shape presented by the opening flap of the bin for disposal of the sharp-perforating material. This last situation culminates in the difficulty of disposing of the medical material used, which often includes large tubular non-metallic components that easily roll up, making it hard to discard the needle that must be turned downwards when deposited in the bin and which is often connected to large metallic and tubular components, a typical example of this being the butterflies. So, when the material is inadequately positioned inside the bin, there is a higher risk of accidental needlestick injuries, and the conventional systems do not allow this disposal procedure to be controlled. The risk of an accidental needlestick injury also increases whenever the amount of sharp material present in the bin exceeds two thirds of its capacity. Now the conventional bins do not have any system designed to prevent the filling thereof beyond the level considered safe.

The present invention is aimed at lowering the risks of direct contact with the bin, of contamination with medical or organic fluids, as well as of imminent needlestick injury and interruption of the clinical procedure, since it allows the safe and effective detachment of the sharp-perforating materials, that is, avoids the contact of the healthcare professional with the sharps and the bin. It also limits the filling capacity of the sharps bin.

### Brief description of the drawings

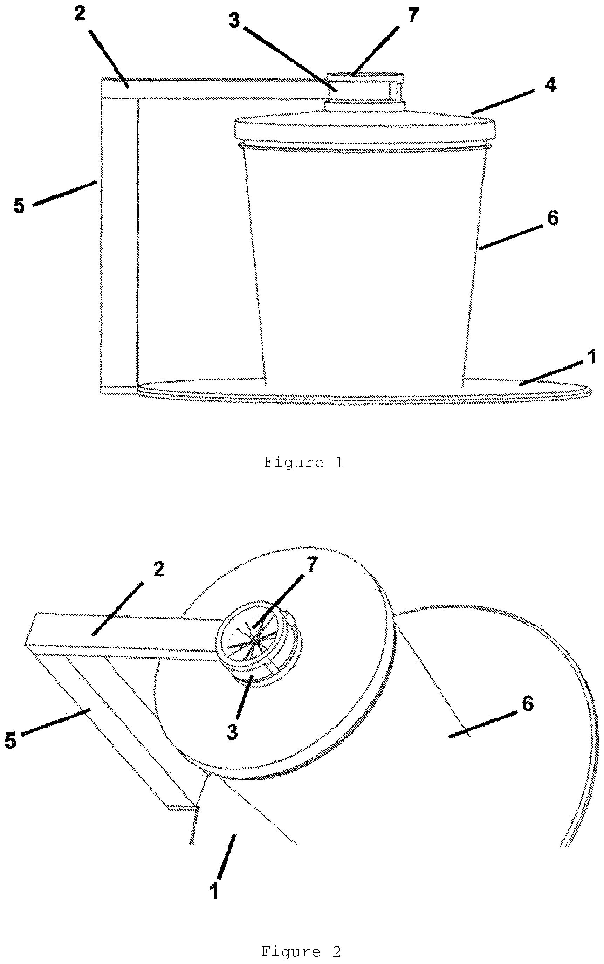

Figures 1 and 2 - Representation of the base (1), wherein is the bin (6) with the lid (4) on it, also showing the arm (2), the claw (3) and the column (5), and at the top of the lid (4), in this illustration, the separating device (7).

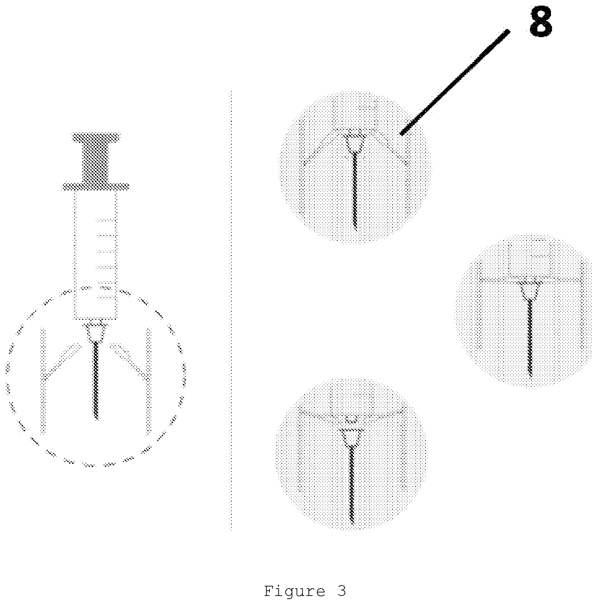

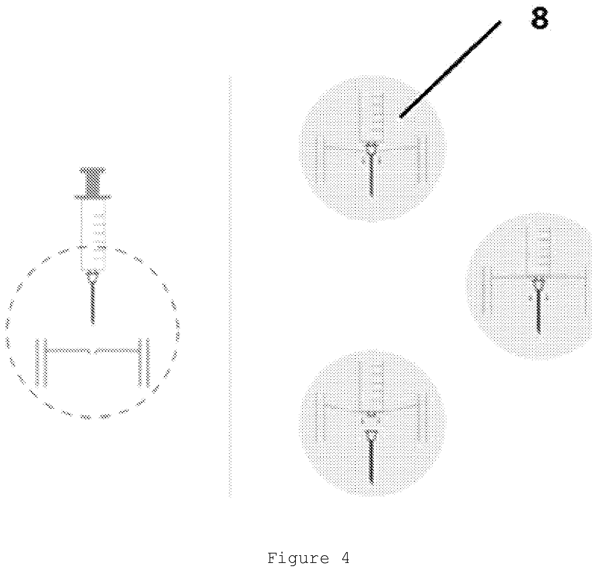

Figures 3 and 4 - representation of the means of retention and extraction (8), performing the downward movement in Figure 3, for the retention, in the case of this illustration, of a needle; and in Figure 4 the upward movement for removal of the needle.

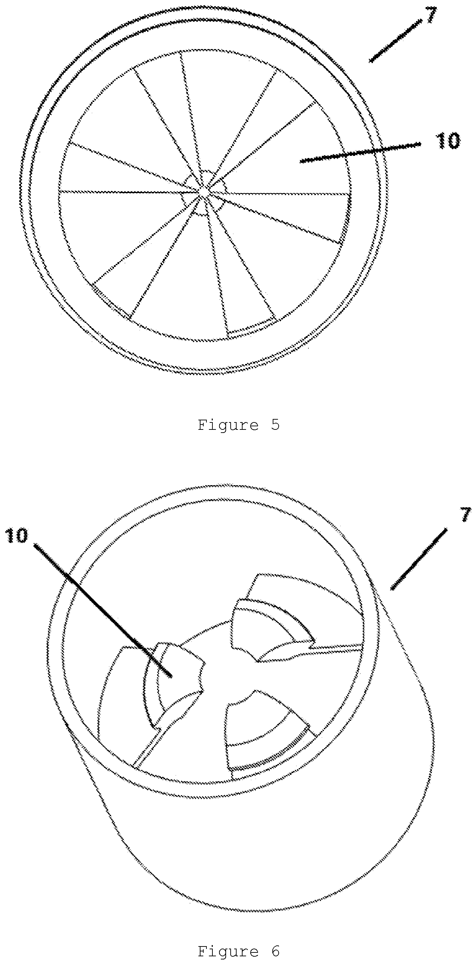

Figures 5 and 6 - representation of the separating device (7) with six and three tabs (10) respectively.

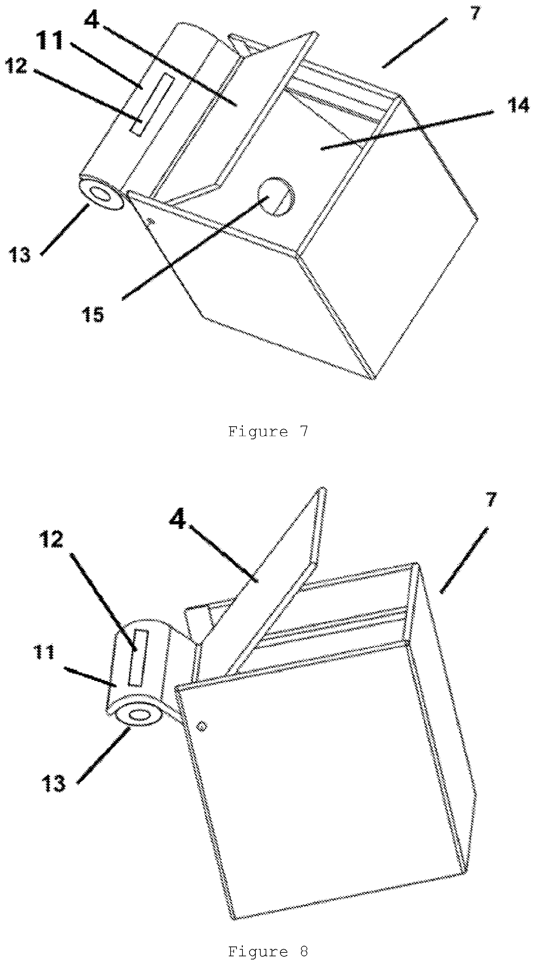

Figures 7 and 8 - representation of the separating device (7) with the flap (11), in which there is the proximity sensor (12) and its power supply (13); also, the connection of the lid (4) to the platform (14) and its hole (15).

### Detailed description of the invention

This invention relates to a system for the removal/disposal and conditioning of sharp-perforating material, the said system being comprised of:
- a base (1) for placing the bin which will be fixed, the said base remaining stable by means of an adjustable claw or "clamps" (3) fixing it to the lid (4) or to the body of the bin (6) presenting different geometries and shapes;
- a column (5) consisting of two tubes which may be rectangular stainless steel tubes, fitted one to the other, to prevent any type of garbage from entering the tangential slot where they are inserted, the one with the largest diameter having at one end the connection to an arm (2), and the one with the smallest diameter being attached to the base (1). It is the largest diameter tube the one which naturally moves vertically, making this column extendable, so that it can adapt to any height of the bin. This vertical movement can be performed through a system of holes and pins or other;
- an arm (2) which has at one end a claw (3) for attachment the lid (4) or bin (6);
- a sharps bin (6) which may have circular, oval, rectangular or another geometric configuration. The bin (6) is provided with a lid (4) having some specific features, such as: fit compatible with the bin; variable height to adjust to the column (5), arm (2) and claw (3); it has such a geometry that it separates the sharp-perforating materials. The said lid (4) can be circular, oval or quadrangular;
- separating devices (7) that allow the disposal of the sharp-perforating material, which can be circular or quadrangular. The separating device (7) allows the bin (6) to be closed when the bin (6) has 2/3 of its capacity filled, this level being visible by means of a mark on the body of the bin (6);
- the separating device (7) incorporated in the lid (4) is intended to discard the sharp-perforating material in order to prevent the risks mentioned above, as well as to accommodate the sharp-perforating material in the bin (6); these separating devices (7) have means of retention and extraction (8) and may also have a cubic configuration suitable for the specificities of sharps with varying geometries and dimensions, such as mandrels, butterflies, etc.

In the separating device (7), with the aim of fighting the risk of contamination, the force exerted by the user in order to successfully separate the sharp-perforating material from the syringe must performed with a downward movement, so that all the residues still present in the sharp-perforating material remain inside the bin.

In the conventional systems, this force is exerted with an upward movement that can lead to the waste inside the syringe being projected or spreading through the workplace, significantly increasing the chances of contamination.

In a preferred embodiment, and through the separating device (7), which is considered the most suitable for the removal of needles from syringes, the means of retention (8) of the said separating device (7) are in the form of tabs (10). In a downward movement, the tabs (10) will retain the needle and then, with another downward movement, a lever movement is created to disconnect the needle from the syringe. Preferably, there are six tabs (10) that provide greater stability in use and help to reduce the strain applied to them, not colliding with each other, but these may be at least two tabs (10). The fact that it comprises a separating device (7) allows the means of retention (8) to retain the needle and to disconnect it from the syringe so that the healthcare professionals do not run the risk of an accidental needlestick injury upon detachment when disposing of the sharps into the bin.

In another preferred embodiment, the force exerted is also downwardly and, as in the previous embodiment, it uses a lever system but requiring only one downward movement, which thus makes it faster to use. In this preferred embodiment, the tabs (10) have a slope, the said slope varying according to the number of tabs (10), in order to reduce the strain exerted on them and providing an increased stability.

Since the geometries and dimensions of the sharp-perforating material are variable, the separating device (7) presents several preferred embodiments. For the disposal of mandrels and butterflies or other materials with variable geometries and dimensions that do not need to be mechanically discarded (separation of needles from syringes), the separating device (7) comprises means of retention (8) which is a flap (11) that has an integrated motion detection sensor (12) with power supply (13), which will automatically open the lid (4) upon the approach of the hand of healthcare professional for the disposal of the material without touching the bin. The lid (4) is provided, in this preferred embodiment, with a platform (14), suitable for placing the butterflies, which can have a hole (15) to place the mandrel. By means of a torsion spring, the lid (4) is forced to remain closed when not in use.

In short, in this invention, the separating device (7) incorporated in the lid (4) has means of retention and extraction (8), the said means of retention and extraction, when moved downwards, retaining the sharp-perforating material and, when moved upwards, the sharp-perforating material is detached and conditioned inside the bin, wherein:
- for syringes, there are means of retention (8), these being at least two tabs (10), with a downward movement retaining the needle and with an upward movement disconnecting the needle from the syringe, and then conditioning the needle inside the bin (6);
- for mandrels, the retention means (8) are a flap (11), a lid (4) with a platform (14) that has a hole (15), and when the flap (11) presents a downward movement, the lid (4) opens for retention of the material in the hole (15) of the platform (14) and when the flap (11) presents an upward movement the material is detached and conditioned inside the bin (6).

The base (1) can have various dimensions and geometries, preferably circular or square, with the arm (2) and column (5) being extendable, which allows the placement of sharp-perforating materials of various dimensions and geometries. Similarly, the claw (3) can also assume varied shapes.

The separating device (7) can be removable or fixed to the lid (4). The lid (4) may have a circular, oval or rectangular shape depending on the geometry of the bin.

In the healthcare sector there is a need for hygienization and asepsis, so the base (1) and the arm (2) and the claw (3) are made of stainless steel having high resistance to oxidation and, preferably, a smooth surface without roughness, compact, without porosity, which facilitates the cleaning and hygienization of the surface, reducing the deposition of microorganisms and making easier the removal thereof. 316L stainless steel is an alloy of iron, chromium and nickel; it is an austenitic steel, which implies a greater resistance to oxidation, easy welding and low magnetic permeability.

Both the bin (6) and the lid (4) are preferably made of polypropylene, but other types of materials can be used. The polypropylene does not present cracking issues and offers excellent mechanical, electrical and chemical resistance to higher temperatures. The bin (6) can preferably have a volume above 3 litres.

This invention allows healthcare institutions to reduce incineration costs, since there is no placement of non-sharps in the bin, thus making it possible to reduce both the volume of material placed and the quantity of bins used.

## Claims

1. A system for removal, extraction and conditioning of sharp-perforating material comprising a base (1) connected to an extendable column (5) and an arm (2), a bin (6) and a lid (4), **characterized in that** further comprises a separating device (7) incorporated in the lid (4) and the referred separating device (7) having means of retention and extraction (8), the said means of retention and extraction (8) when moved downwards retaining the sharp-perforating material, and, when moved upwards, disconnecting and conditioning the sharp-perforating material inside the bin (6) .

2. A system according to the previous claim, **characterized in that** the means of retention (8) are at least two tabs (10), the said tabs (10) performing:
a. a downward movement, retaining the sharp-perforating material; and
b. an upward movement, disconnecting the sharp-perforating material and conditioning it inside the bin (6).

3. A system according to the previous claim, **characterized in that** the tabs (10) are six.

4. A system according to claim 1, **characterized in that** the means of retention (8) are a flap (11) a platform (14), the said platform (14) being connected to the lid (4) and having a hole (15), and **in that** those means of retention (8) are configured that when the flap (11) presents a downward movement, the lid (4) opens for retention of the material in the hole (15) of the platform (14) and when the flap (11) presents an upward movement the material is detached and conditioned inside the bin (6).

5. A system according to claim 1, **characterized in that** the tab (11) integrates a motion detection sensor (12) with power supply (13), which opens the lid (4) when identifying the approach of an object.

6. A system according to the previous claims, **characterized in that** the column (5) being comprised of two tubes fitted one to the other, the largest diameter tube having a vertical movement and at one end the connection to an arm (2), and the smallest diameter tube being fixed to the base (1).

7. A system according to the previous claims, **characterized in that** the arm (2) has at one end a claw (3) for attachment to the lid (4) or to the bin (6).

8. A system according to the previous claims, **characterized in that** the separating device (7) can be removable or fixed to the lid (4).

9. A system according to the previous claims, **characterized in that** the base (1), the arm (2) and the claw (3) are made of stainless steel.

10. A system according to the previous claims, **characterized in that** the base (1), the arm (2) and the claw (3) have a smooth surface, without roughness, compact and with no pores.

11. A system according to the previous claims, **characterized in that** the bin (6) and the lid (4) are made of polypropylene.

12. A system according to the previous claims, **characterized in that** the bin (6) has a volume higher than 3 litres.
